(19) Europäisches Patentamt
European Patent Office
Office européen des brevets

(11) **EP 0 583 280 B1**

(12) **EUROPÄISCHE PATENTSCHRIFT**

(45) Veröffentlichungstag und Bekanntmachung des Hinweises auf die Patenterteilung:
07.08.1996 Patentblatt 1996/32

(21) Anmeldenummer: 92909055.3

(22) Anmeldetag: 22.04.1992

(51) Int. Cl.$^6$: **A61K 31/64**

(86) Internationale Anmeldenummer:
PCT/EP92/00884

(87) Internationale Veröffentlichungsnummer:
WO 92/19245 (12.11.1992 Gazette 1992/28)

(54) **VERWENDUNG VON TORASEMID ZUR BEHANDLUNG VON HIRNÖDEMEN**

USE OF TORASEMIDE FOR THE TREATMENT OF CEREBRAL OEDEMA

UTILISATION DU TORASEMIDE POUR LE TRAITEMENT D'OEDEMES CEREBRAUX

(84) Benannte Vertragsstaaten:
AT BE CH DE DK ES FR GB GR IT LI LU NL SE

(30) Priorität: 27.04.1991 DE 4113820

(43) Veröffentlichungstag der Anmeldung:
23.02.1994 Patentblatt 1994/08

(73) Patentinhaber: BOEHRINGER MANNHEIM GMBH
D-68298 Mannheim (DE)

(72) Erfinder:
• BOELKE, Tim
D-6800 Mannheim 1 (DE)
• KLING, Lothar
D-6800 Mannheim 1 (DE)
• KOENIG, Reinhard
D-6718 Grünstadt (DE)

(74) Vertreter: Weber, Manfred, Dr.
c/o Boehringer Mannheim GmbH,
Patentabteilung,
Sandhoferstrasse 116
68298 Mannheim (DE)

(56) Entgegenhaltungen:
EP-A- 0 338 415

• Neurosurgery, Band 4, Nr. 1, 1979, P.A. TORNHEIM et al.: "Effect of furosemide on experimental traumatic cerebral edema", Seiten 48-52
• Neurology, Band 24, Juli 1974, R.A. CLASEN et al.: "Furosemide and pentobarbital in cryogenic cerebral injury and edema", Seiten 642-648.
• DRUGS, Band 41, Nr. 1, Januar 1991, H.A. FRIEDEL et al.: "Torasemide. A review of its pharmacological properties and therapeutic potential", Seiten 81-103.
• J. Neurochem., (12. Meet. of the Intern. Soc. for Neurochem., Algarve, 23. - 28. April 1989), Band 52(Suppl.), 1989, B. MASCIEEL et al.: "New tornarmide derivatives inhibit brain alices and antrocytra swelling induced by potarrium", Seite 183.
• Deutsche Medizinische Wochenschrift, Band 99, Nr. 18, 3. Mai 1974, J. THILMANN et al.: "Untersuchungen zur Behandlung des Hirnödems mit hohen Dosen Furosemid", Seiten 932-935.
• J.E.F. REYNOLDS: "Martindale - The Extra Pharmacopoeia", 29. Auflage, 1989, Seite 1003, Pharmaceutical Press, London, GB, siehe Seite 1003, Nr. 1272-d: "Torasemide"
• Zentralblatt für Neurochirurgie. 50(1989) 142-144

**Beschreibung**

Gegenstände der Erfindung sind die Verwendung von Torasemid zur Herstellung von Arzneimitteln für die Behandlung dar Schwellung von Nerven- und Gliazellen bei Hirnödemen, welche als Folge von z.B. Schädel-Hirn-Traumen und Metastasen auftreten, und oral und parenteral applizierbares Torasemid.

Torasemid, chemische Bezeichnung 1-Isopropyl-3-[(4-m-toluidino-3-pyridyl)-sulfonyl]-harnstoff ist bekannt als Schleifendiuretikum, bei dessen systemischer Gabe Urinvolumen und Elekrolytausscheidung mit dem Logarithmus der Torasemid-Dosis linear ansteigt.

In in-vitro Untersuchungen konnte gezeigt werden, daß Torasemid die azidose-induzierte Schwellung von C6 Gliomzellen deutlich vermindert. Bei in-vivo Untersuchungen an Ratten wurde darüber hinaus festgestellt, daß Torasemid den intrakranialen Druck (ICP) beim zytotoxischen Hirnödem, ausgelöst durch hyposmolare Hyperhydratation, zu senken vermag.

Eine Azidose tritt im Gehirn bei zerebraler Ischämie, bei Schädel-Hirntraumen, bei epileptischen Anfällen, sowie unter anderen pathophysiologischen Bedingungen auf. Bei zerebraler Ischämie kann der pH-Wert auf 6,5 bis 6,0 abfallen, unter hyperglykämischen Bedingungen kann die Azidose noch ausgeprägter sein. Als Folge der Azidose und des damit verbundenen verringerten Energiestoffwechsels findet ein Nettoeinstrom von $Na^+$-und $Cl^-$-Ionen in die Zellen statt, wobei die intrazelluläre Erhöhung der Osmolarität zu einer entsprechenden Wasserbewegung und damit zu einer Zellschwellung führt.

Für Diuretika, wie z.B. Bumetanid und Furosemid, die in der Niere wie Torasemid über die Hemmung des NaCl-KCl-Cotransports wirken, wurde gezeigt, daß sie einen experimentell erhöhten intrakranialen Druck nicht zu beeinflussen vermögen (s. den Artikel " Effect of furosemide, bumetanide and mannitol on intracranial pressure in experimental brain edema of the rat " von C. Plangger u. H. Völkl, veröffentlicht in Zent bl Neurochir 50: 142-144, 1989). Insofern war es überraschend, daß unter Torasemid eine Senkung des erhöhten intrakranialen Druckes beobachtet werden konnte.

Die Wirksamkeit von Torasemid bei der Verminderung der Schwellung von Gliazellen bzw. bei der Senkung des ICP wird durch die nachstehend näher beschriebenen, durch Figuren veranschaulichten Untersuchungen belegt.

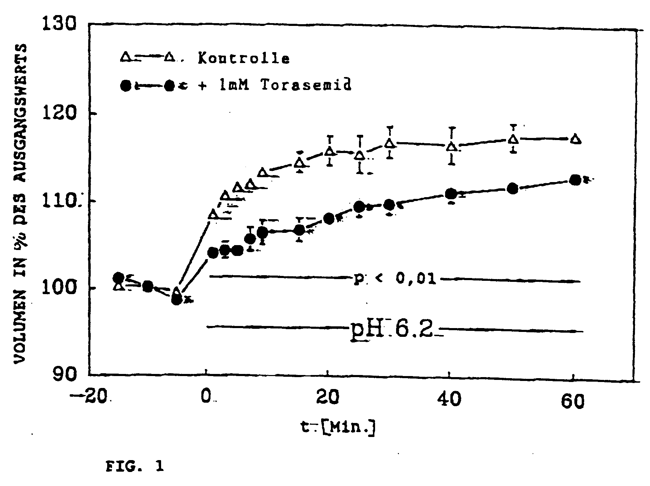

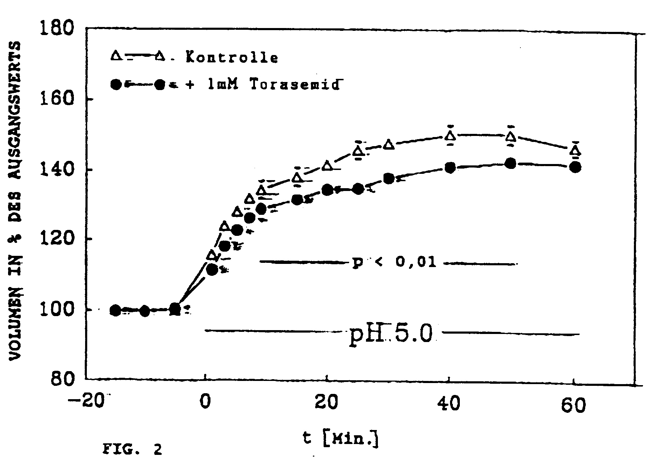

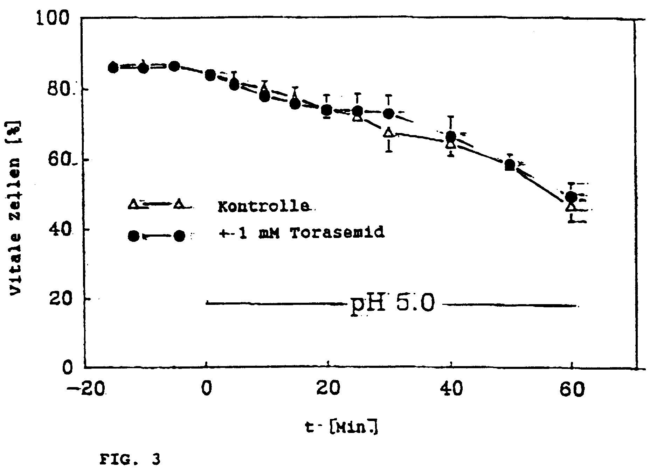

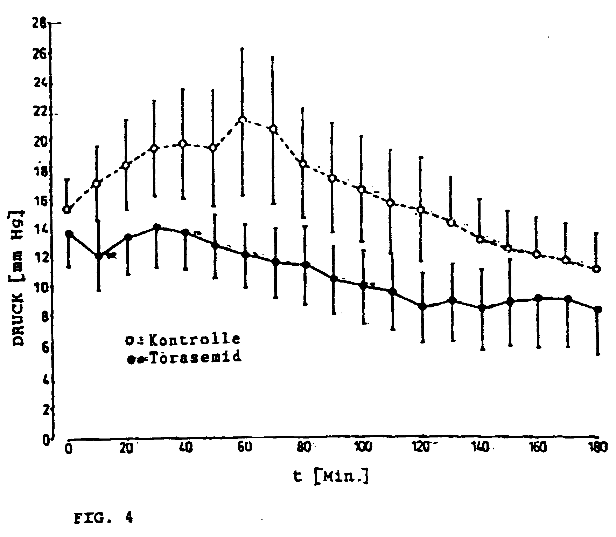

Die Fig. 1 und 2 zeigen den zeitlichen Verlauf einer in-vitro azidose-induzierten Zellschwellung von C6 Gliomzellen nach Inkubation mit Torasemid bzw. bei Kontollversuchen ohne Verumzugabe. Die zeitliche Veränderung der Vitalität der C6 Gliomzellen bei diesen Versuchen ist in der Fig. 3 dargestellt. Die Fig. 4 bis 6 zeigen die zeitlichen Veränderungen des ICP, des mittleren systemischem arteriellen Blutdrucks und des zerebralen Perfusionsdrucks bei Patten nach Induzierung eines Hirnödems in zwei Versuchsreihen, wobei in der einen Versuchsreihe Torasemid und in der anderen (Kontroll)-Versuchsreihe isotone Kochsalzlösung intravenös appliziert-wurde.

Bei den-in-vitro Untersuchungen zum Einfluß von Torasemid auf azidose-induzierte Zellschwellung wurden C6 Gliomzellen mit gliaspezifischen Eigenschaften verwendet. Die Gliazellen wuchsen als Monolayer in Petrischalen unter konventionellen Kulturbedingungen bei 95% Raumluft, 5% $CO_2$ und einer Temperatur von 37°C. Kulturmedium war Dulbecco's Minimal Essential Medium (DMEM) mit 25 mM Bikarbonat als Puffer. Das Medium enthielt außerdem 10% fötales Kälberserum (FKS) sowie 100 IU/ml Penizillin G und 50 µg/ml Streptomyzin. Zur Durchführung eines Versuchs wurden die Zellen aus 6 Kulturschalen mit Trypsin geerntet, zweimal in FKS-freiem Medium gewaschen und anschließend in eine Versuchskammer eingebracht. Die Kammer gewährleistete eine homogene Stabilität der Einzelsuspension über mehrere Stunden, sowie die Kontrolle des Milieus durch kontinuierliche Registrierung des pH-Werts, des Sauerstoffpartialdrucks und der Temperatur. Die Bestimmung des Zellvolumens erfolgte quantitativ mit Hilfe eines Durchflußzytometers nach dem Coulter-Verfahren. Das Gerät-verwendete zudem hydrodynamische Fokussierung der Partikel zur-Verbesserung der Meßgenauigkeit. Damit können Veränderungen des Zellvolumens von < 1% sicher erfaßt werden. Die Vitalität der Zellen wurde durchflußzytometrisch durch Ausschluß von Propidiumiodid bestimmt.

Versuchsdurchführung: Zunächst wurden in einer Kontrollphase (45 min) das Zellvolumen sowie die Zell-Vitalität auf ihren konstanten Verlauf überprüft. Außerdem wurde die Osmolarität des Mediums bestimmt. Darüber hinaus wurde in Parallelversuchen festgestellt, daß bei ausschließlicher Zugabe von Torasemid keine signifikante Veränderung des Zellvolumens und keine Beeinflussung der Vitalität der C6 Gliomzellen stattfand. Die Zellschwellung wurde nach der Kontrollphase durch Absenken des pH-Wertes auf 6,2 bzw. 5,0 mit Hilfe isotoner Milchsäure erzeugt. Gleichzeitig wurde der pCO2 der Kammer auf 80 bis 100 mm Hg angehoben. Die Versuchsdauer betrug 60 Minuten. Bei pH 6,2 und 5,0 wurden je 2 parallele Versuche durchgeführt. Bei jeweils einem Versuch wurde 15 Minuten vordem Ansäuern Torasemid in einer Endkonzentration von 1 mM dem Medium zugegeben. Der andere Versuch diente als Kontrolle.

Die Ergebnisse sind in den Fig. 1 und 2 aufgeführt, in welchen das Zellvolumen (in % des Ausgangswerts) über die Versuchsdauer aufgetragen ist. Außerdem ist jeweils die Zeitspanne angegeben, während der der pH-Wert auf 6,2 bzw. 5,0 abgesenkt war. Die Zeitspanne zwischen den beiden Versuchen, innerhalb derer die unter Torasemid erhaltenen Versuchsdaten signifikant (p < 0,01) unterschieden waren von der Kontrolle, sind ebenfalls markiert. Die in den Kurven aufgetragenen Zellvolumenwerte sind arithmetische Mittel ($\bar{x}$) ± SEM. Die Ergebnisse zeigen deutlich die Hemmung der Zellschwellung durch Torasemid. Wie die Fig. 3 zeigt, hat außerdem Torasemid per se keinen Einfluß auf die Vitalität. von C6 Gliomzellen. In der Fig. 3 ist die Veränderung der Vitalität der C6 Gliomzellen über die Versuchsdauer dargestellt. Außerdem ist die Zeitspanne markiert, während der der pH-Wert auf 5,0 abgesenkt war. Man ersieht aus den Kurven, daß zwar die pH-Absenkung aber nicht die Torasemid-Zugabe einen Einfluß auf die Vitalität hat. Die Ergebnisse der beschriebenen Versuche wurden mit Hilfe der Varianzanalyse und dem Kruskal-Wallis-Test für den Intergruppenvergleich auf Signifikanz analysiert.

Die Wirkung von Torasemid beim Hirnödem wurde in vivo im pharmakologischen Modell des zytotoxischen Hirnödems der Ratte untersucht. Bei 150 bis 240 g schweren, männlichen Ratten wurde zur kontinuierlichen Erfassung des systemischen arteriellen Blutdrucks ein Katheder in die rechte Arteria femoralis eingeschoben und mittels eines Gould P10 EZ Blutdruckumwandlers, eines Siemens-Monitors und eines 2-Kanal-Recorders (Linseis L 650) gemessen und aufgezeichnet. Zur kontinuierlichen Erfassung des ICP wurde durch eine Trepanation der linken Schädelvorderseite ein Wick-Katheter eingeführt. Der ICP wurde mittels eines elektromagnetischen Statham-Umwandlers P 23 Db, der mit einem Hellige-Elektromanometer verbunden war, und eines 2-Kanal-Recorders (Linseis L 650) gemessen und aufgezeichnet. Um einen möglichen diuretischen Effekt auszuschließen, wurden die Ratten funktionell nephrektomiert.

Versuchsdurchführung: Zur Induzierung des Hirnödems wurden 100 ml Aqua bidest./kg KG mit 0,5 ml/min in die rechte Vena jugularis infundiert. Hiernach wurden 100 mg Torasemid/kg KG in 10 ml Flüssigkeit/kg KG und bei einem Paralielversuch 10 ml isotonische Kochsalzlösung (Placebo)/kg KG injiziert. ICP und Blutdruck wurden 3 Stunden lang nach Gabe von Torasemid oder Placebo kontinuierlich aufgezeichnet. 6 Ratten erhielten eine Torasemid-Injektion, 7 Ratten erhielten Placebo.

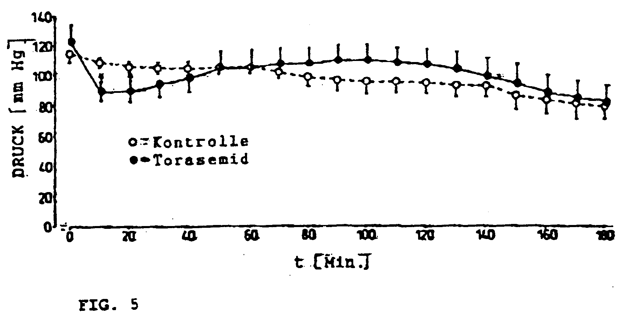

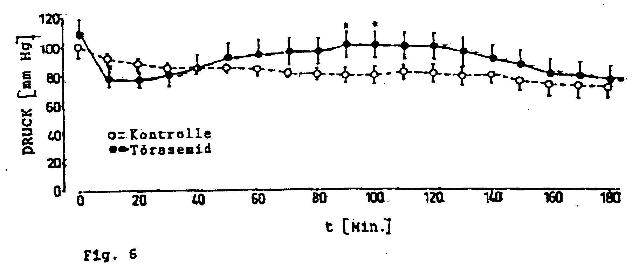

Die ausgewerteten Ergebnisse der ICP- und Blutdruckmessungen sind in den Fig. 4 bzw. 5 aufgeführt. Die Fig. 4 zeigt, daß der ICP bei den mit Torasemid behandelten Ratten zu jedem Zeitpunkt niedriger war als bei den in Parallelversuch mit Placebo behandelten Tieren. Die Unterschiede waren jedoch statistisch nicht signifikant. Zur Erhärtung des Befunds wurde deshalb noch der zerebrale Perfusionsdruck bestimmt (der zerebrale Perfusionsdruck ist gleich der Differenz zwischen dem systemischen mittleren arteriellen Blutdruck und dem ICP). Dabei ergab sich (s. Fig. 6), daß dessen Werte 90 und 100 Minuten nach der Gabevon Torasemid signifikant höher waren als in der Kontrollgruppe. Dies war hauptsächlich auf die viel niedrigeren ICP-Werte bei den mit Torasemid behandelten Ratten zurückzuführen. In den Fig 4 bis 6 sind arithmetische Mittelwerte ($\overline{x}$) ± SEM gegen die Zeit aufgetragen. Die Prüfung auf signifikante Unterschiede zwischen den mit Torasemid und Placebo behandelten Versuchsgruppen erfolgte mittels eines einseitigen student t-tests für ungepaarte Daten.

Zusammenfassend läßt sich sagen: Sowohl die in-vitro Experimente mit C6 Gliomzellen als auch die in-vivo Versuche an Ratten zeigen, daß Torasemid bei induzierter Schwellung der Gliazellen und erhöhtem ICP schwellungshemmend bzw. druckmindernd wirkt. Torasemid ist auch deshalb günstig, weil - wies bei den Versuchen mit C6 Gliomzellen (s.o.) festgestellt worden ist - Torasemid dies Vitalität der Gliazellen nicht beeinträchtigt; diese wird in der gleichen Versuchsanordnung z.B. durch Amilorid vermindert. Mögliche Ursachen für die mit Torasemid und nicht mit z.B. Furosemid und Bumetanid beobachtete pharmakologische Wirkung ist die hohe Lipophilie von Torasemid (Oktanol/$H_2O$-Koeffizient: 3,71)

Die Herstellung des Torasemids erfolgt nach dem in der Patentschrift DE 25 16 025 C2 beschriebenen Verfahren.

Torasemid läßt sich aufgrund seiner hohen Bioverfügbarkeit in äquivalenten Dosen sowohl oral als auch parenteral zur Therapie von Hirnödemen verschiedener Genese einsetzen.

Zur Herstellung von oral zu verabreichenden Arzneimitteln wird Torasemid in an sich bekannter Weise mit geeigneten pharmazeutischen Trägersubstanzen, Aroma-, Geschmacks- und Farbstoffen gemischt, und beispielsweise als Tabletten oder Dragees ausgeformt oder unter Zugabe entsprechender Hilfsstoffe in Wasser oder Öl, wie z.B. Olivenöl, suspendiert oder gelöst.

Zur Herstellung eines parenteral zu verabreichenden pharmazeutischen Präparats wird Torasemid, gegebenenfalls in Form seiner pharmakologisch unbedenklichen Salze, in an sich bekannter Weise unter Zugabe entsprechender Hilfsstoffe, wie z.B. Emulgatoren, in Wasser suspendiert oder gelöst.

Toresamid wird in Mengen zwischen 5 und 40 mg pro Tag appliziert.

Eine beispielhafte Rezeptur für eine Tablette mit 10 mg Wirkstoff setzt sich wie folgt zusammen:

| | |
|---|---|
| Torasemid | 10,0 mg |
| Laktose . 1 $H_2O$ | 116,0 mg |
| Maisstärke | 32,0 mg |
| kolloidales Siliciumdioxid | 1,2 mg |
| Magnesiumstearat | 0,4 - 1,o mg |
| Gewicht einer Tablette | 159,6 - 160,2 mg |

Eine beispielhafte Rezeptur für ein parenteral zu verabreichendes pharmazeutisches Präparat mit 10 mg Wirkstoff setzt sich wie folgt zusammen:

| Natrium-Torasemid | 10,631 mg |
|---|---|
| Natriumhydroxid | 0,05 mg |
| Trometamol (2-Amino-2-hydroxymethyl-1,3-propandiol) | 0,25 mg |
| Macrogol (Polyethylenglykole) | 225,00 mg |
| Wasser | 1804,069 mg |
| Stickstoff | Q.s. |

**Patentansprüche**

1.  Verwendung von 1-Isopropyl-3-[(4-m-toluidino-3-pyridyl)-sulfonyl]-harnstoff oder dessen pharmakologisch unbedenkliche Salze zur Herstellung von Arzneimitteln für die Behandlung von Hirnödeman.

2.  Verwendung nach Anspruch 1 zur Herstellung von Arzneimitteln für die Behandlung von Hirnödemen, welche als Folge von schweren Schädel-Hirntraumen, zerebraler Ischämie, Schlaganfall, Metastasen oder epileptischen Anfällen auftreten.

3.  Verwendung nach Anspruch 1 zur Herstellung von Arzneimitteln für die Behandlung von durch hypoosmolare Hyperhydratation ausgelösten zytotoxischen Hirnödemen.

**Claims**

1.  Use of 1-isopropyl-3-[(4-m-toluidino-3-pyridyl)-sulphonyl]-urea or of its pharmacologically acceptable salts for the production of medicaments for the treatment of brain oedemas.

2.  Use according to claim 1 for the production of medicaments for the treatment of brain oedemas which occur as a result of severe skull-brain traumas, cerebral ischaemia, strokes, metastases or epileptic fits.

3.  Use according to claim 1 for the production of medicaments for the treatment of cytotoxic brain oedemas initiated by hypoosmolar hyperhydration.

**Revendications**

1.  Utilisation de la 1-isopropyl-3-[(4-m-toluidino-3-pyridyl)-sulfonyl]-urée ou des ses sels pharmacologiquement acceptables, pour la préparation de médicaments destinés au traitement d'oedèmes cérébraux.

2.  Utilisation selon la revendication 1, pour la préparation de médicaments destinés au traitement d'oedèmes cérébraux survenus à la suite de traumatismes crâniens graves, d'ischémie cérébrale, de crises d'apoplexie, de métastases ou de crises d'épilepsie.

3.  Utilisation selon la revendication 1, pour la préparation de médicaments destinés au traitement d'oedèmes cérébraux cytotoxiques survenus à la suite d'hyperhydratation hypo-osmolaire.

FIG. 1

FIG. 2

FIG. 3

FIG. 4

FIG. 5

Fig. 6